# EUROPÄISCHE PATENTSCHRIFT

(11) **EP 1 961 436 B1**
(45) Veröffentlichungstag und Bekanntmachung des Hinweises auf die Patenterteilung: **24.08.2016**
(21) Anmeldenummer: 07003826.0
(22) Anmeldetag: 24.02.2007
(51) Int. Cl.: A61M 5/148, A61B 5/145, A61M 5/145, A61M 5/168, A61M 5/142

(54) **Infusionssystem**
Infusion system
Système d'infusion

(43) Veröffentlichungstag der Anmeldung: 27.08.2008
(73) Patentinhaber: Roche Diabetes Care GmbH, 68305 Mannheim (DE); F. Hoffmann-La Roche AG, 4070 Basel (CH)
(72) Erfinder: Haar, Hans-Peter, 69168 Wiesloch (DE)
(74) Vertreter: Twelmeier, Ulrich

(56) Entgegenhaltungen:
- WO-A-92/16990
- DE-U1- 20 017 371
- US-A- 4 573 994
- US-A1- 2003 100 864
- US-B1- 6 416 495

## Beschreibung

Die Erfindung betrifft ein Infusionssystem zum Verabreichen eines flüssigen Medikaments. Ein Infusionssystem mit den im Oberbegriff des Anspruchs 1 angegebenen Merkmalen ist aus der DE 200 17 371 U1 bekannt. Eine Pumpeneinheit für ein derartiges Infusionssystem ist auch aus der US 2003/0100864 A1 bekannt.

Aus der US 6 416 495 ist ein Implantat zum Verabreichen flüssiger Medikamente bekannt. Das Implantat weist eine Pumpe mit einem einen Akkumulator zur Energieversorgung und einem wieder auffüllbaren Speichervolumen für das zu verabreichende Medikament auf.

Viele Diabetiker sind zur Regulierung ihres Blutglucosespiegels auf externe Insulingaben angewiesen. Üblicherweise erfolgen diese Insulingaben mehrmals täglich in Form von Injektionen. Aus medizinischer Sicht sind jedoch kontinuierliche Insulingaben vorteilhaft, wie sie beispielsweise mit dem aus der WO 2004/098390 A2 bekannten Infusionssystem möglich sind. Nachteilig an bekannten Infusionssystemen sind einerseits ein von vielen Benutzem als unzureichend empfundener Tragekomfort und andererseits erhebliche Kosten, welche die Kosten einer Behandlung durch mehrmals tägliche Insulininjektionen bei weitem übersteigen.

Aufgabe der Erfindung ist es deshalb einen Weg aufzuzeigen, wie die Kosten eines Infusionssystems gesenkt und der Benutzerkomfort erhöht werden kann.

Diese Aufgabe wird erfindungsgemäß durch ein Infusionssystem mit den im Anspruch 1 angegebenen Merkmalen gelöst. Vorteilhafte Weiterbildungen der Erfindung sind Gegenstand von Unteransprüchen.

Das erfindungsgemäße Infusionssystem umfasst eine Kanülenträgereinheit und eine daran anschließbare Pumpeneinheit, die bestimmungsgemäß zusammen von einem

Benutzer unter der Kleidung getragen werden. Bei einem erfindungsgemäßen Infusionssystem können die Pumpeneinheit und die Kanülenträgereinheit derart aneinander angepasst sein, dass nach einem Anschließen der Pumpeneinheit an die Kanülenträgereinheit die Pumpeneinheit an der Kanülenträgereinheit anliegt, jedoch ist dies nicht zwingend erforderlich. Beispielsweise kann die Pumpeneinheit auch von der Kanülenträgereinheit beabstandet am Körper getragen und ein Schlauch als Verbindungsleitung genutzt werden. Indem die Kanülenträgereinheit und die Pumpeneinheit unter der Kleidung getragen werden könnnen, wird ein hoher Tragekomfort erreicht, da das zu verabreichende Medikament von der Pumpeneinheit auf einem kurzen Weg über die Infusionskanüle der anliegenden Kanülenträgereinheit in den Körper des Patienten eingeleitet werden kann. Lange Infusionsschläuche, die von den meisten Benutzern als lästig und störend empfunden werden, können auf diese Weise vermieden werden. Zudem bergen lange Infusionsschläuche auch das Risiko, dass der Flüssigkeitstransport durch mechanische Bewegung und Belastung gestört wird.

Der modulare Aufbau eines erfindungsgemäßen Infusionssystems ermöglicht dabei eine kosteneffiziente Realisierung. Das Speichervolumen der Pumpeneinheit eines erfindungsgemäßen Infusionssystems kann nämlich immer wieder neu mit dem zu verabreichenden Medikament aufgefüllt und diese somit mehrfach verwendet werden. Die Herstellungskosten einer Kanülenträgereinheit mit der zum Einstich in den Körper eines Patienten bestimmten Infusionskanüle betragen typischerweise nur einen Bruchteil der Herstellungskosten einer Pumpeneinheit. Die Kanülenträgereinheit kann deshalb ohne wesentliche Erhöhung der Systemkosten als hygienischer Wegwerfartikel gestaltet werden.

Zur Energieversorgung der Pumpeneinheit ist bei dem erfindungsgemäßen Infusionssystem ein Akkumulator vorgesehen, der mit einer an die Pumpeneinheit angepassten Ladestation aufgeladen werden kann. Bevorzugt erfolgt das Aufladen des Akkumulators durch induktive Kopplung mit der Ladestation. Auf diese Weise können nämlich eventuelle Leckagestellen, durch die Feuchtigkeit oder Schmutz in die Pumpeneinheit eindringen könnte, auf ein Minimum reduziert werden.

Wie bereits erwähnt, hat die Pumpeneinheit ein Speichervolumen für das zu verabreichende Medikament. Bevorzugt beträgt dieses Speichervolumen weniger als 2 ml, besonders bevorzugt weniger als 1,5 ml und insbesondere nicht mehr als 1 ml. Diese Maßnahme hat den scheinbaren Nachteil, dass der in dem Speichervolumen speicherbare Medikamentenvorrat beispielsweise bei einer Verwendung des Infusionssystems zum Verabreichen von Insulin für einen Diabetiker nur für etwa einen halben Tag oder sogar nur für einige Stunden ausreicht.

Wenn bei einer Pumpeneinheit eines erfindungsgemäßen Infusionssystems der Medikamentenvorrat erschöpft ist, kann das Speichervolumen wieder aufgefüllt werden. Die Kanülenträgereinheit kann dabei am Körper des Patienten verbleiben, so dass das aufwändige und für Patienten schmerzhafte Setzen einer neuen Infusionskanüle vermieden werden kann. Bei einem erfindungsgemäßen Infusionssystem kann eine Infusionskanüle deshalb trotz des kleinen Speichervolumens der Pumpeneinheit über eine längere Zeit z. B. drei bis vier Tagen verwendet werden und dabei im Körper des Patienten verbleiben.

Prinzipiell kann die Pumpeneinheit derart ausgestaltet sein, dass das Auffüllen des Speichervolumens ohne Abkoppeln von der Kanülenträgereinheit möglich ist, beispielsweise indem ein in dem Speichervolumen enthaltener Medikamentenbehälter ausgetauscht wird. Bevorzugt wird die Pumpeneinheit jedoch von der Kanülenträgereinheit abgekoppelt, wenn das in dem Speichervolumen enthaltene Medikament verabreicht wurde. Nach dem Auffüllen des Speichervolumens kann die Pumpeneinheit wieder an die Kanülenträgereinheit angekoppelt werden. Besonders günstig ist es in diesem Zusammenhang, wenn ein Patient zwei Pumpeneinheiten abwechselnd benutzt, so dass unmittelbar nach dem Abkoppeln einer Pumpeneinheit, deren Speichervolumen erschöpft ist, eine Pumpeneinheit mit einem gefüllten Speichervolumen an die Kanülenträgereinheit angeschlossen werden kann.

Bei dem erfindungsgemäßen Infusionssystem kann durch ein kleines Speichervolumen der Pumpeneinheit somit erreicht werden, dass ein Patient nur ein relativ kleines und kompaktes Gerät am Körper tragen muss. Dies ist für den Tragekomfort außerordentlich günstig und wiegt den scheinbaren Nachteil eines häufigeren Auffüllens der Pumpeneinheiten bei weitem auf. Mit einem erfindungsgemäßen Infusionssystem kann deshalb trotz verhältnismäßig geringer Kosten ein außerordentlich hoher Benutzerkomfort erreicht werden.

Ein bevorzugtes Ausführungsbeispiel eines erfindungsgemäßen Infusionssystems sieht vor, dass die Ladestation zum Aufladen des Tanks mit dem zu verabreichenden Medikament eingerichtet ist. Auf diese Weise kann ein Benutzer sowohl den Akkumulator der Pumpeneinheit als auch deren Speichervolumen ohne große Mühe aufladen. Besonders günstig ist es dabei, eine Ladestation in Kombination mit zwei Pumpeneinheiten zu nutzen, die abwechselnd am Körper getragen werden, so dass stets eine Pumpeneinheit an die Ladestation angekoppelt ist und dort aufgeladen wird. Ein wichtiger Aspekt der Erfindung betrifft deshalb ein Gerätset für ein erfindungsgemäßes Infusionssystem mit zwei Pumpeneinheiten, die jeweils eine Pumpe, einen Akkumulator zur Energieversorgung der Pumpe und ein Speichervolumen für das zu verabreichende Medikament umfassen sowie mit einer Ladestation, an die jeweils eine Pumpeneinheit zum Aufladen des Akkumulators und dem Auffüllen der Ladestation angekoppelt werden kann.

Ein alternatives Ausführungsbeispiel eines erfindungsgemäßen Infusionssystems sieht vor, dass das Speichervolumen der Pumpeneinheit zur Aufnahme von zwei Medikamentenbehältern ausgebildet ist. Auf diese Weise kann von der Pumpeneinheit zunächst der Inhalt des ersten Medikamentenbehälters und anschließend der Inhalt des zweiten Medikamentenbehälters verabreicht werden. Sobald der erste Medikamentenbehälter leer ist, kann ein Benutzer diesen gegen einen vollen Behälter austauschen. Der Vorteil einer Pumpeneinheit mit einem Speichervolumen zur Aufnahme von zwei Medikamentenbehältem im Gegensatz zu einer Pumpeneinheit mit einem Speichervolumen zur Aufnahme eines größeren Medikamentenbehälters, besteht darin, dass der Benutzer den Zeitpunkt des Auswechselns eines leeren Behälters in einem Zeitfenster von mehreren Stunden frei wählen kann. Ist der erste Behälter leer, hat ein Benutzer bequem Zeit, diesen gegen einen vollen Behälter auszutauschen, da es genügt den Austausch vorzunehmen, bevor auch der zweite Behälter leer ist. Wird dagegen eine Pumpeneinheit mit nur einem einzigen Medikamentenbehälter verwendet, muss der Austausch entweder unmittelbar nach dem vollständigen Entleeren des Behälters vorgenommen werden oder aber teuerer Wirkstoff verschwendet werden, indem ein nur teilweise entleerter Behälter gegen einen vollen ausgetauscht wird. Ein Aspekt der Erfindung der auch selbstständige Bedeutung haben kann, betrifft deshalb eine Pumpeneinheit für ein Infusionssystem, wobei die Pumpeneinheit zur Aufnahme von zwei austauschbaren Medikamentenbehältern ausgebildet ist.

Weitere Einzelheiten und Vorteile werden anhand von Ausführungsbeispielen unter Bezugnahme auf die beigefügten Zeichnungen erläutert. Gleiche und einander entsprechende Bauteile sind dabei mit übereinstimmenden Bezugszeichen gekennzeichnet. Die im folgenden erläuterten Merkmale der Ausführungsbeispiele können einzeln und in Kombination zum Gegenstand von Ansprüchen gemacht werden.

Es zeigen:
- Figur 1: eine schematische Darstellung eines Ausführungsbeispiels eines erfindungsgemäßen Infusionssystems mit einer Kanülenträgereinheit, einer Pumpeneinheit und einer Ladestation;
- Figur 2: eine Seitenansicht eines Ausführungsbeispiels einer Pumpeneinheit und eines Ausführungsbeispiels einer dazu passenden Kanülenträgereinheit;
- Figur 3: die in Figur 2 dargestellte Kanülenträgereinheit in einer Schrägansicht;
- Figur 4: ein weiteres Ausführungsbeispiel einer Pumpeneinheit mit einem Ausführungsbeispiel einer angeschlossenen Kanülenträgereinheit in einer Schnittansicht;
- Figur 5: eine Detailansicht zu Figur 4;
- Figur 6: die in Figur 4 dargestellte Ausführungsbeispiel einer Pumpeneinheit mit einem Ausführungsbeispiel einer daran angeschlossenen Ladestation in einer Schnittansicht;
- Figur 7: eine Schrägansicht zu Figur 6;
- Figur 8: eine Explosionsdarstellung zu Figur 7;
- Figur 9: eine Detailansicht eines Pumpenkolbens beim Auspressen von Medikamentenflüssigkeit;
- Figur 10: eine Detailansicht eines weiteren Ausführungsbeispiels eines Medikamentenbehälters des Infusionssystems;
- Figur 11: ein weiteres Ausführungsbeispiel eines Mechanismus zum Anschließen eines Medikamentenbehälters an die Flüssigkeitsleitung der Kanülenträgereinheit oder Ladestation;
- Figur 12: ein weiteres Ausführungsbeispiel einer Pumpeneinheit und einer dazu passenden Kanülenträgereinheit.

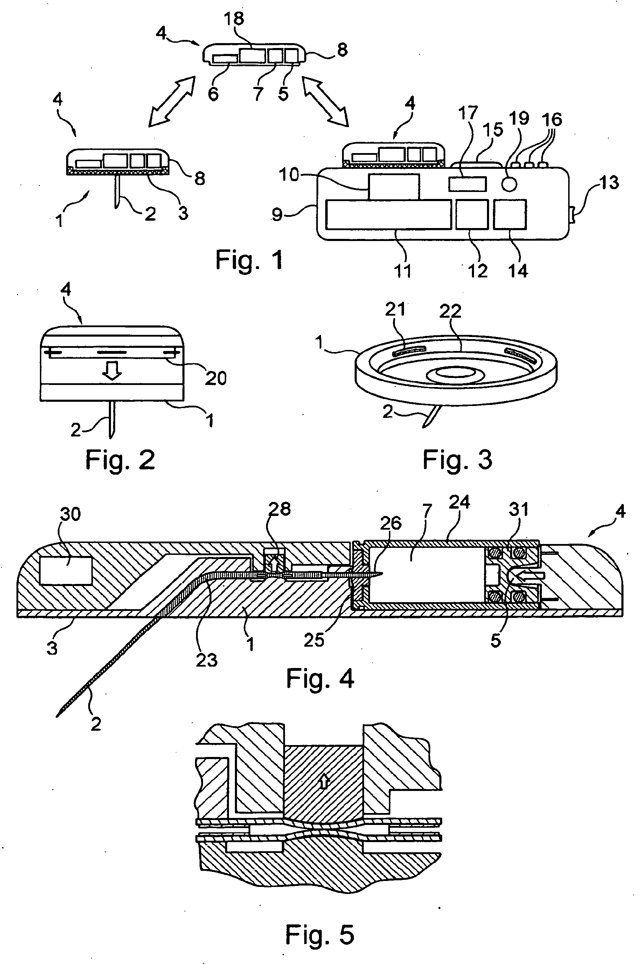

Figur 1 zeigt eine schematische Darstellung eines Ausführungsbeispiels eines Infusionssystems zum Verabreichen eines flüssigen Medikaments, beispielsweise Insulin. Zu dem Infusionssystem gehört eine Kanülenträgereinheit 1, die bestimmungsgemäß am Körper eines Patienten befestigt wird und eine Infusionskanüle 2 zum Einstich in den Körper des Patienten aufweist. Das dargestellte Ausführungsbeispiel der Kanülenträgereinheit 1 hat eine Auflagefläche 3, beispielsweise ein Kissen, die an ihrer Unterseite mit Klebstoff beschichtet sein kann, um leichter am Körper eines Patienten befestigt werden zu können.

Bei der Infusionskanüle 2 kann es sich beispielsweise um eine Hohlnadel handeln, die mit einem spitzen Ende in den Körper des Patienten eingestochen wird und anschließend zum Einleiten des flüssigen Medikaments dient. Die Infusionskanüle 2 kann aber auch ein flexibles Kunststoffröhrchen, beispielsweise aus Teflon, sein, das mit einer harten Hilfs- oder Trägerkanüle in den Körper eingeführt wird. Die Hilfs- oder Trägerkanüle kann beispielsweise aus Stahl sein und einen V-förmigen Querschnitt haben, so dass die flexible Infusionskanüle beim Einstich in einer von der Hilfs- oder Trägerkanüle gebildeten Rinne liegt. Die spitze Hilfs- oder Trägerkanüle kann nach dem Einführen der flexiblen Infusionskanüle 2 entfernt werden.

Zu dem Infusionssystem gehört ferner eine Pumpeneinheit 4, die formschlüssig an die Kanülenträgereinheit 1 anschließbar ist. Das dargestellte Ausführungsbeispiel der Pumpeneinheit 4 enthält eine Pumpe 5, einen Akkumulator 6 zur Energieversorgung der Pumpe 5 und ein Speichervolumen 7 für das zu verabreichende Medikament. Wie Figur 1 zeigt, weist die Pumpeneinheit 4 ein Gehäuse 8 auf, das nach dem Anschließen der Pumpeneinheit 4 an die Kanülenträgereinheit 1 an der Kanülenträgereinheit 1 anliegt.

Durch Verbinden der Pumpeneinheit 4 mit einer Kanülenträgereinheit 1 entsteht eine kleine und kompakte Vorrichtung, die von einem Patienten bequem am Körper getragen werden kann und ihm ein flüssiges Medikament verabreicht. Der Weg von dem Speichervolumen der Pumpeneinheit 1 zu der Kanüle ist dabei vorteilhaft kurz und beträgt allenfalls wenige Zentimeter. Bei einer ersten Inbetriebnahme nach dem Anbringen der Kanülenträgereinheit am Körper des Patienten muss deshalb nur ein sehr kleines Luftvolumen aus einer von dem Speichervolumen 7 zu der Infusionskanüle 2 führenden Flüssigkeitsleitung (siehe Fig. 4) verdrängt werden, so dass selbst bei einer geringen Pumpenleistung schon nach sehr kurzer Zeit das Medikament in den Körper des Patienten eingeleitet werden kann.

Die Pumpeneinheit 4 kann von einer am Körper des Patienten befestigten Kanülenträgereinheit 1 abgenommen werden und zum Aufladen ihres Akkumulators 6 und zum Wiederbefüllen ihres Speichervolumens 7 an eine ebenfalls zu dem Infusionssystem gehörende Ladestation 9 angekoppelt werden. Ein Ausführungsbeispiel der Ladestation 9 ist schematisch ebenfalls in Figur 1 dargestellt.

Ein erhöhter Benutzerkomfort lässt sich dabei dadurch erreichen, dass ein Patient zwei Pumpeneinheiten 4 nutzt, die abwechselnd an die Kanülenträgereinheit 1 und die Ladestation 9 angeschlossen werden. Auf diese Weise wird eine Pumpeneinheit 4 von der Ladestation 9 wieder aufgeladen, während die andere Pumpeneinheit 4 an eine Kanülenträgereinheit 1 angeschlossen ist und zur Infusion genutzt wird.

Das Speichervolumen 7 der Pumpeneinheit 4 beträgt weniger als 2 ml, vorzugsweise weniger als 1,5 ml, insbesondere nicht mehr als 1 ml. Auf diese Weise kann die Pumpeneinheit besonders klein und kompakt ausgeführt werden, was einen wichtigen Vorteil darstellt, da die Pumpeneinheit 4 bestimmungsgemäß zusammen mit einer Kanülenträgereinheit von einem Patienten unter der Kleidung am Körper getragen wird und möglichst wenig auffallen sollte. Ein kleineres Speichervolumen 7 macht zwar ein häufigeres Auffüllen mit dem zu verabreichenden Medikament erforderlich, jedoch ist dies bei dem dargestellten Infusionssystem nur ein scheinbarer Nachteil, da sich die Pumpeneinheit 4 leicht von der Kanülenträgereinheit 1 abkoppeln und durch eine gefüllte Pumpeneinheit 4 ersetzen lässt.

Der in der Pumpeneinheit 4 enthaltene Akkumulator 6 ist für einen Benutzer unzugänglich in der Pumpeneinheit 4 eingebaut, beispielsweise in das Gehäuse 8 der Pumpeneinheit 4 integriert. Diese Maßnahme hat den Vorteil, dass sich potentielle Leckagestellen, durch die Feuchtigkeit oder Schmutz in die Pumpeneinheit eindringen könnten minimiert lassen. Besonders gut geeignet sind insbesondere Lithium-Polymer-Akkumulatoren, die als flexible Folienbeutel ausgebildet und deshalb leicht an die Form eines in der Pumpeneinheit 4 zur Verfügung stehenden Einbauraumes angepasst werden können. Mit Lithium-Polymer-Akkumulatoren lässt sich die Pumpeneinheit 4 deshalb besonders kompakt ausführen. Um den Akkumulator 6 und damit die Pumpeneinheit 4 so klein und kompakt wie möglich ausbilden zu können, hat der Akkumulator 6 bevorzugt nur eine Energiespeicherkapazität für etwa 4 bis 12 Stunden, beispielsweise von 500 Joule, bevorzugt nicht mehr als 200 Joule.

Das in Figur 1 schematisch dargestellte Ausführungsbeispiel einer Ladestation 9 hat ein Aufnahmefach 10 für einen Vorratsbehälter mit dem zu verabreichenden Medikament. Zum Auffüllen des Speichervolumens 7 einer an die Ladestation 9 angekoppelten Pumpeneinheit 4 wird Medikament aus einem in das Aufnahmefach 10 eingelegten Vorratsbehälter in das Speichervolumen 7 der Pumpeneinheit gepumpt. Prinzipiell kann zu diesem Zweck die Pumpe 5 der angekoppelten Pumpeneinheit 4 verwendet werden; bevorzugt hat die Ladestation 9 jedoch hierfür eine eigene Pumpe 11.

Die Ladestation 9 enthält einen leistungsstarken Energiespeicher 12, beispielsweise eine austauschbare Batterie oder bevorzugt einen Akkumulator, der zum Aufladen des Akkumulators 6 der Pumpenstation 4 elektrische Energie an den Akkumulator 6 der Pumpenstation 4 abgibt. Auf diese Weise kann die Ladestation 9 von einem Patienten beispielsweise in einer Jacken- oder Handtasche mitgeführt und auch unterwegs genutzt werden. Die Speicherkapazität des Energiespeichers 12 ist bevorzugt mindestens 10 mal so groß wie die des Akkumulators 6 der Pumpeneinheit 4, so dass die Ladestation mehrere Tage, beispielsweise 5 Tage, ohne Nachladen genutzt werden kann.

Die Ladestation 9 hat ferner einen Steckverbinder 13 zum Anschluss an ein Stromnetz, so dass ein in der Ladestation 9 enthaltener Akkumulator 12 über das öffentliche Stromnetz aufgeladen werden kann, beispielsweise über Nacht. Der Energiespeicher 12 der Ladestation 9 kann austauschbar in einem benutzerzugänglichen Batteriefach angeordnet sein. Bevorzugt ist der Energiespeicher 12 aber ein benutzerunzugänglich in der Ladestation 9 eingebauter Akkumulator 9. Für einen hohen Benutzerkomfort ist es ein wichtiger Vorteil, dass die Ladestation 9 auch unterwegs, also ohne Zugang zu einem Stromnetz, zum Aufladen des Akkumulators 6 der Pumpenstation 4 genutzt werden kann. Die Ladestation 9 ist deshalb dafür eingerichtet, die Energie zum Aufladen des Akkumulators 6 einer angekoppelten Pumpeneinheit 4 dem internen Energiespeicher 12 zu entnehmen.

In die Ladestation 9 kann ein Messgerät 14 zur Messung einer Analytkonzentration einer Körperflüssigkeitsprobe, insbesondere zur Bestimmung des Glucosegehalts, integriert sein. Das Messgerät 14 kann beispielsweise fotometrisch oder elektrochemisch den Glucosegehalt einer auf einen Teststreifen aufgebrachten Körperflüssigkeitsprobe, beispielsweise Blut oder interstitieller Flüssigkeit, bestimmen und einem Benutzer mit einer Anzeigeeinrichtung 15, beispielsweise einer Flüssigkristall-Anzeige, anzeigen. Derartige Messgeräte sind einem Fachmann geläufig und werden beispielsweise von Diabetikern zur Messung ihres Blutzuckerspiegels benutzt, so dass weitere Erläuterungen zum Aufbau des in die Ladestation 9 integrierten Messgeräts 14 nicht erforderlich sind.

Um den Benutzerkomfort für Diabetiker weiter zu erhöhen, kann in die Ladestation 9 zusätzlich ein Stichgerät integriert werden, mit dem sich ein Patient stechen kann, beispielsweise in den Finger, um eine Körperflüssigkeitsprobe zu gewinnen, die anschließend mit dem Messgerät 14 untersucht werden kann.

Die Ladestation 9 hat benutzerbetätigbare Bedienungselemente 16, beispielsweise Tasten, mit denen ein Benutzer das in die Ladestation 9 integrierte Messgerät 15 bedienen und insbesondere auch Steuerbefehle zur Steuerung der Pumpeneinheit 4 eingeben kann.

Die Ladestation 9 kommuniziert im Betrieb drahtlos mit der bzw. den Pumpeneinheiten 4 des Infusionssystems. Die Ladestation 9 weist zu diesem Zweck einen Sender 17 und die Pumpenstation 4 einen Empfänger 18 auf. Um die Vorteile einer bidirektionalen Kommunikation nutzen zu können, haben die Pumpenstationen ebenfalls einen Sender und die Ladestation auch einen Empfänger, beispielsweise als Bluetooth-Schnittstelle. Durch Verwendung charakteristischer Kennzeichnungscodes kann erreicht werden, dass die Ladestation 9 gezielt eine individuelle Pumpeneinheit 4, beispielsweise die derzeit vom Patienten zusammen mit einer Kanülenträgereinheit 1 am Körper getragene Pumpeneinheit 4, ansprechen und mit ihr Informationen austauschen kann. Auf diese Weise kann die Infusionsrate, mit der die Pumpeneinheit 4 das flüssige Medikament an den Patienten abgibt, von einem Benutzer über die Ladestation 9 vorgegeben werden. Möglich ist es auch, dass die Ladestation von dem integrierten Messgerät 15 ermittelte Messergebnisse zur Glucosekonzentration oder einer anderen Analytkonzentration einer Körperflüssigkeitsprobe mit einer Steuereinheit, beispielsweise in Form eines Mikroprozessors, auswertet und die Infusionsrate in Abhängigkeit von diesen Messergebnissen steuert.

Eine weitere Möglichkeit für die Kommunikation zwischen der bzw. den Pumpeneinheiten 4 und der Ladestation 9 besteht darin, dass ein Datentransfer von der Pumpeneinheit 4 zur Ladestation 9 durch das Ansetzten der Pumpeneinheit 4 an die Ladestation 9 ausgelöst wird. Auf diese Weise kann das energieintensive Senden von Daten einer am Körper getragenen Pumpeneinheit 4 an eine Ladestation 9 vermieden und die Pumpeneinheit 4 besonders kompakt ausgeführt werden. Beispielsweise kann auf Sender und Empfänger in der Pumpeneinheit 4 verzichtet werden und zur Datenübertragung ein Kontaktfeld genutzt werden, das beim Aufladen der Pumpeneinheit 4 mit einem Kontaktfeld der Ladestation 9 in elektrischen Kontakt tritt und so den Anschluss einer Datenleitung bewirkt. Möglich ist es auch die Datenübertragung zwischen Pumpeneinheit 4 und Ladestation 9 induktiv oder mittels Infrarotstrahlung vorzunehmen.

Die Pumpeneinheit 4 enthält einen oder mehrere Sensoren zur Überwachung ihres Betriebszustandes. Eine mögliche Ausführungsform sieht vor, dass die Pumpeneinheit 4 ihren Betriebszustand an die Ladestation 9 meldet, so dass beispielsweise Fehlfunktionen oder ein niedriger Ladezustand des Akkumulators oder des Speichervolumens einem Benutzer durch ein Warnsignal angezeigt werden können. Bevorzugt hat die Ladestation 9 deshalb eine akustische Signaleinrichtung 19 zum Erzeugen von Signaltönen. Alternativ oder zusätzlich kann auch die Pumpeneinheit 4 mit einer Signaleinrichtung, insbesondere einer akustischen Signaleinrichtung, ausgestattet werden, um einen Benutzer gegebenenfalls auf eine Fehlfunktion und/oder einen niedrigen Ladezustand aufmerksam zu machen. Die Pumpeneinheit kann insbesondere auch einen Sensor zum Überwachen des Füllstands des Speichervolumens 7 aufweisen. Wenn der Füllstand unter einen vorgegebenen Schwellenwert absinkt, kann dies einem Benutzer signalisiert werden, indem die Pumpeneinheit 4 ein entsprechendes Signal erzeugt. Ein solches Signal kann von der Pumpeneinheit beispielsweise als akustisches Signal, das für einen Benutzer hörbar ist, erzeugt werden. Möglich ist es aber auch, dass die Pumpeneinheit 4 ein elektromagnetisches Signal an die Ladestation 9 sendet, die einem Benutzer den niedrigen Füllstand durch ein akustisches Signal oder ein Signallicht anzeigt.

Die Ladestation 9 kann ferner einen elektronischen Speicher haben, in dem Benutzerdaten, insbesondere Messergebnisse und verabreichte Infusionsmengen gespeichert werden können. Das beschriebene Infusionssystem kann mit Injektionssystemen, beispielsweise im Handel erhältlichen Insulin Pens (stiftförmige Injektionsgeräte zur Verabreichung von Insulin) kombiniert werden, um einen vorübergehenden Spitzenbedarf abzudecken. Die Ladestation 9 kann hierfür mit einer Aufnahme für ein Injektionsgerät, insbesondere einen Insulin Pen, ausgestattet sein, so dass das Injektionsgerät in der Ladestation mit dem zu injizierenden Wirkstoff und/Energie aufgeladen werden kann.

Möglich ist es insbesondere auch, die Ladestation 9 als Datenverteiler eines Systems zur Behandlung chronischer Krankheiten, beispielsweise Diabetes, zu nutzen. Beispielsweise kann die Ladestation dafür eingerichtet sein, mit einem separaten Messgerät, insbesondere einem Blutzuckermessgerät, und/oder einem separaten Injektionsgerät, beispielsweise einem Insulin Pen, Daten auszutauschen. Dabei können für die Behandlung wichtige Daten verschiedener Systemkomponenten, insbesondere Messergebnisse und Infusionsdaten, in der Ladestation 9 gesammelt und gespeichert werden. Die Ladestation 9 kann diese Daten selbst auswerten oder aber, was bevorzugt ist, an ein Auswertegerät, beispielsweise einen PC, weitergeben. Die Auswertung der Daten kann insbesondere auch in einem separaten Messgerät, beispielsweise einem Blutzuckermessgerät erfolgen. Dabei ist es nicht erforderlich alle wichtigen Daten in Ladestation 9 zu sammeln, da in dem Messgerät selbst wichtige Daten erzeugt werden, die von Ladestation 9 nicht benötigt werden.

Die Ladestation 9 kann insbesondere auch zum Aufladen von Akkumulatoren anderer Systemkomponenten, beispielsweise Messgerät und/oder Injektionsgerät, dienen und dabei Daten von diesen Systemkomponenten, die insbesondere auch eigene Datenspeicher aufweisen könnne, erhalten oder diesen liefern.

Figur 2 zeigt ein Ausführungsbeispiel einer Kanülenträgereinheit 1 sowie ein Ausführungsbeispiel einer daran anschließbaren Pumpeneinheit 4 des im vorhergehenden beschriebenen Infusionssystems. Figur 3 zeigt die in Figur 2 dargestellte Kanülenträgereinheit 1 in einer Schrägansicht. Die Pumpeneinheit 4 wird formschlüssig, bei dem dargestellten Ausführungsbeispiel durch eine Drehbewegung, an die Kanülenträgereinheit 1 angeschlossen. Die Ankopplung der Infusionskanüle 2 an das Speichervolumen 7 der Pumpeneinheit 4 kann beispielsweise in der in der WO 2004/026375 A1 beschriebenen Weise durch ein Drehverbindung mit diskreten Rastpositionen vorgenommen werden. Geeignet ist insbesondere ein Schraub- oder Bajonettverschluss. Die Pumpeneinheit 4 hat zu diesem Zweck eines oder mehrere in Figur 2 dargestellte Verbindungselemente 20, die mit einem oder mehreren in Figur 3 dargestellten Verbindungselementen 21 der Kanülenträgereinheit 1 zusammenwirken. Bei dem in den Figuren 2 und 3 dargestellten Ausführungsbeispiel bilden die Pumpeneinheit 4 und die Kanülenträgereinheit 1 ein Infusionsgerät mit einem flüssigkeitsdichten Gehäuse.

Zwischen der Pumpeneinheit 4 und einer angeschlossenen Kanülenträgereinheit 1 wirkt eine Dichtung 22, die beispielsweise als ein in Figur 3 dargestellter Dichtring ausgebildet sein kann, der an der Kanülenträgereinheit 1 befestigt sein kann.

Figur 4 zeigt ein weiteres Ausführungsbeispiel einer Pumpeneinheit 4 mit einem daran angeschlossenen Ausführungsbeispiel einer Kanülenträgereinheit 1 des im vorhergehenden beschriebenen Infusionssystems. Die Kanülenträgereinheit 1 hat eine Infusionskanüle 2 zum Einstich in den Körper des Patienten sowie eine Auflagefläche 3, die bestimmungsgemäß auf die Haut eines Patienten aufgelegt wird. Ist die Kanülenträgereinheit 1, wie in Figur 4 dargestellt, an eine Pumpeneinheit 4 angeschlossen, führt eine Flüssigkeitsleitung 23 von der Infusionskanüle 2 zu dem Speichervolumen 7 der Pumpeneinheit 4, in dem sich das zu verabreichende Medikament befindet. Bei dem dargestellten Ausführungsbeispiel ist diese Flüssigkeitsleitung 23 Teil der Kanülenträgereinheit 1. Das Speichervolumen 7 der Pumpeneinheit 4 ist bei dem dargestellten Ausführungsbeispiel zur Aufnahme einer austauschbaren Medikamentenampulle 24 ausgebildet. Diese Medikamentenampulle 24 ist mit einem Septum 25 verschlossen, dass beim Anschließen der Pumpeneinheit 4 an die Kanülenträgereinheit 1 von einer Transferkanüle 26 der Kanülenträgereinheit 1, die an dem Ende der Flüssigkeitsleitung 23 angeordnet ist, durchstochen wird. Moderne Septen können ohne weiteres viele Male von einer Kanüle durchstochen werden ohne undicht zu werden.

Das Speichervolumen zur Aufnahme eines Medikamentenbehälters auszubilden, der in der Ladestation mehrfach aufgefüllt werden kann, hat den Vorteil, dass dieser Medikamentenbehälter bei Bedarf ausgetauscht werden kann, beispielsweise bei Auftreten von Verschleißerscheinungen. Prinzipiell kann das Speichervolumen 7 der Pumpeneinheit 4 aber auch ein fest eingebauter Tank sein.

Die Pumpeneinheit 4 weist einen Drucksensor 28 zum Überwachen eines Infusionsdrucks des flüssigen Medikaments auf. Dieser Infusionsdruck wird bei dem dargestellten Ausführungsbeispiel in der Flüssigkeitsleitung 23 gemessen. Der Drucksensor 28 ist dabei derart ausgebildet, dass ein von dem Infusionsdruck bewirktes Aufblähen eines Schlauchabschnitts, durch den bestimmungsgemäß das zu verabreichende Medikament von dem Speichervolumen 7 zu der Infusionskanüle 2 einer Kanülenträgereinheit 1 strömt, erfasst wird. Der Sensor 28 ist schematisch in Figur 5, die eine Detailansicht zu Figur 4 zeigt, dargestellt.

Figur 5 zeigt den von einem Schlauch gebildeten Abschnitt der Flüssigkeitsleitung 23, dessen Aufblähen von dem Drucksensor 28 erfasst wird. Bei dem dargestellten Ausführungsbeispiel umfasst der Drucksensor 28 ein Tastglied, das bei einem Aufblähen des Schlauchabschnitts in Richtung des in Figur 5 dargestellten Pfeils verschoben wird. Diese Verschiebung wird erfasst und in ein elektrisches Sensorsignal umgewandelt. Der Drucksensor 28 könnte ein Aufblähen des Flüssigkeitsschlauchs beispielsweise auch durch eine kapazitive Messung erfassen. Hierfür kann eine Außenfläche des Schlauches mit einer gegenüberliegenden Sensorfläche des Drucksensors einen Kondensator bilden, dessen Kapazität sich beim Aufblähen des Schlauchabschnitts ändert.

Das von dem Drucksensor 28 erzeugte Sensorsignal wird von einer in der Pumpeneinheit 4 enthaltenen Steuerungselektronik 30 ausgewertet. Die Steuerungselektronik 30 erzeugt in Abhängigkeit von Druckdaten, die aus dem Sensorsignal ermittelt werden, Steuersignale zur Steuerung der Pumpe 5. Eine Verstopfung der Flüssigkeitsleitung 23, beispielsweise durch ein Blutgerinsel am Ausgang der Infusionskanüle 2, kann auf diese Weise durch einen ansteigenden Flüssigkeitsdruck erkannt werden. Erreicht der Flüssigkeitsdruck eine kritische Obergrenze, kann die Pumpe 4 von der Steuerungselektronik 30 abgeschaltet und ein Warnsignal an die Ladestation 9 gesendet werden. Die Ladestation 9 erzeugt dann ein Warnsignal, um einen Benutzer auf das kritische Ansteigen des Infusionsdrucks hinzuweisen, so dass geeignete Gegenmaßnahmen ergriffen werden können.

Die Pumpe 5 der Pumpeneinheit 4 hat einen Kolben 31, der das Speichervolumen 7, das beispielsweise als Tank oder, wie bei dem dargestellten Ausführungsbeispiel, zur Aufnahme einer Ampulle 24 ausgebildet sein kann, eingeführt wird, um das Medikament auszupressen. Geeignete Pumpen sind beispielsweise aus der WO 01/72358 A1 oder DE 100 48 220 A1 bekannt.

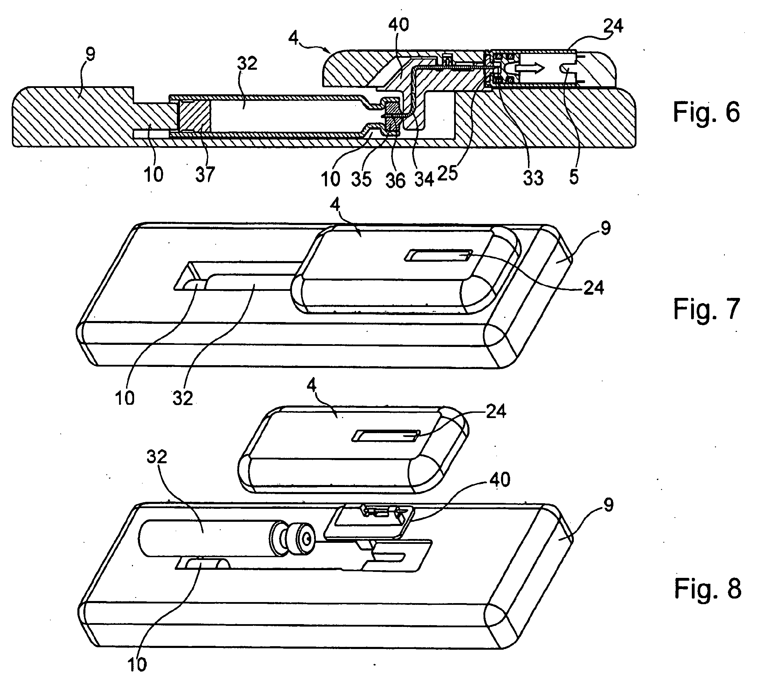

Figur 6 zeigt die im vorhergehenden beschriebene Pumpeneinheit 4 zusammen mit einem Ausführungsbeispiel einer dazugehörenden Ladestation 9, an welche die Pumpeneinheit 4 angeschlossen ist. Figur 7 zeigt eine schematisch Schrägansicht zu Figur 6, Figur 8 eine Explosionsdarstellung zu Figur 7. Die Ladestation 9 hat ein Aufnahmefach 10 für einen Vorratsbehälter 32 mit dem zu verabreichenden Medikament. Das Volumen des Vorratsbehälters 32 wird bevorzugt so gewählt, dass das Speichervolumen 7 der Pumpeneinheit 4 damit mindestens dreimal, bevorzugt vier bis sechs Mal gefüllt werden kann. Bei dem dargestellten Ausführungsbeispiel ist der Vorratsbehälter 32 eine Ampulle mit einem Volumen von 3,2 ml, so dass das Speichervolumen 7 der Pumpeneinheit 4, das beispielsweise 0,8 ml beträgt, vier Mal gefüllt werden kann.

Bei dem dargestellten Ausführungsbeispiel wird der in die Ladestation 9 eingesetzte Vorratsbehälter 32 in der selben Weise an das Speichervolumen 7 der Pumpenstation 4 angekoppelt, wie die zum Einstich in den Körper des Patienten bestimmte Infusionskanüle 2 der Kanülenträgereinheit 1, nämlich indem mit einer Transferkanüle 33, die am Ende einer Flüssigkeitsleitung 34 angeordnet ist, das Septum 25 einer in das Speichervolumen 7 eingesetzten Ampulle 24 durchstochen wird. In entsprechender Weise wird auch das Septum 35 eines in die Ladestation 9 eingesetzten Vorratbehälters 32 mit einer Transferkanüle 36 durchstochen, so dass der Vorratsbehälter 9 über die Flüssigkeitsleitung 34 an das Speichervolumen 7 der Pumpeneinheit 9 angeschlossen ist.

Die Ladestation 9 enthält eine Pumpe 11, die zum Befüllen des Speichervolumens 7 einer angeschlossenen Pumpeneinheit 4 Flüssigkeit aus dem eingesetzten Vorratsbehälter 32 auspresst. Die Pumpe 11 der Ladestation 9 kann nach dem selben Prinzip wie die Pumpe 5 der Pumpeneinheit 4 aufgebaut sein, also einen Kolben 31 aufweisen, mit dem ein Stopfen 37 in den Vorratsbehälter 32 hinein geschoben wird, um darin enthaltene Flüssigkeit auszupressen.

Die zum Befüllen des Speichervolumens 7 der Pumpeneinheit 4 verwendete Flüssigkeitsleitung 34 entspricht in ihrem Aufbau der Flüssigkeitsleitung 23 der Kanülenträgereinheit 1, so dass mit dem Drucksensor 28 der Pumpeneinheit 4 auch der Flüssigkeitsdruck beim Auffüllen des Speichervolumens 7 überwacht werden kann. Aus dem Sensorsignal des Drucksensors 28 ermittelte Druckdaten werden von der Pumpeneinheit 4 an eine Steuereinheit der Ladestation 9 übermittelt, die die Pumpe 10 der Ladestation 9 in Abhängigkeit von den erhaltenen Druckdaten steuert und bei einer Fehlfunktion ein Warnsignal erzeugt, damit ein Benutzer bei Bedarf korrigierend eingreifen kann.

Bei dem in den Figuren 6 bis 8 dargestellten Ausführungsbeispiel ist die Flüssigkeitsleitung 34 der Ladestation 9 ebenso wie die Transferkanülen 33, 36 Teil eines austauschbaren Transfermoduls 40 der Ladestation 9. Diese Maßnahme hat den Vorteil, dass die relativ verschleißanfällige Transferleitung 34 mit den Transferkanülen 33, 36 bei Bedarf mit geringen Aufwand ausgetauscht werden kann.

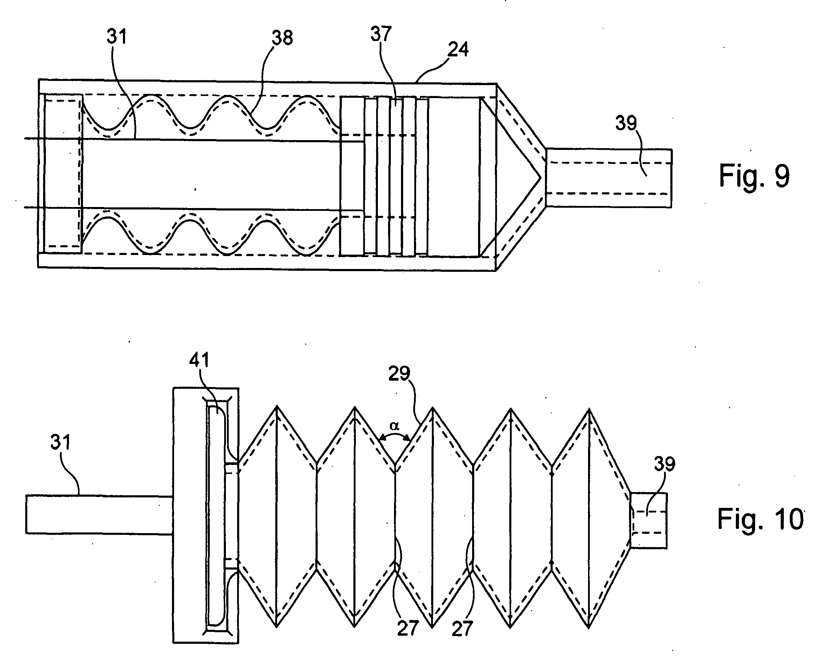

In Figur 9 ist ein Ausführungsbeispiel einer in das Speichervolumen 7 der Pumpeneinheit 4 eingesetzten Medikamentenampulle 24 in einer Detailansicht dargestellt. Die wieder befüllbare Ampulle 24 zur Speicherung eines Medikaments hat ein vorderes Ende mit einer Öffnung 39 zum Durchtritt des Medikaments und ein hinteres Ende, das mit einem verschiebbaren Stopfen 37 verschlossen ist. Der Stopfen 37 wird zum Auspressen des in der Ampulle 24 enthaltenen Medikaments von dem Kolben 31 in die Ampulle 24 hinein geschoben. An dem Stopfen 37 ist ein Balg 38 als Sterilschutzhülle angebracht, der den Kolben 31 umgibt und die Innenwände der Ampulle 24 vor Kontamination schützt. Bei dem dargestellten Ausführungsbeispiel ist der Balg 38 flüssigkeitsdicht an dem hinteren Ende der Ampulle 24 und an dem Stopfen 37 befestigt, beispielsweise durch Verkleben.

Die Pumpeneinheit 4 kann auch zur Aufnahme eines Medikamentenbehälters ausgebildet sein, der nicht als Ampulle ausgebildet ist. Beispielsweise kann der Medikamentenbehälter 24 auch als Schlauchbeutel oder Tube ausgebildet sein. Anstelle einer Pumpe 7, die wie bei dem dargestellten Ausführungsbeispiel zum Auspressen des Medikaments einen Kolben 31 in eine Ampulle 24 hinein schiebt, kann bei Verwendung anderer Medikamentenbehälter beispielsweise eine Rollen- oder Stempelpumpe verwendet werden.

Eine weitere vorteilhafte Möglichkeit besteht darin, den Medikamentenbehälter als Balgbeutel 29 gemäß Figur 10 auszubilden. Bei dem Balgbeutel handelt es sich um einen wieder befüllbaren Schlauchbeutel, der an seinem vorderen Ende eine Öffnung 39 zum Durchtritt des Medikaments aufweist und ähnlich wie ein Faltenbalg umlaufende Falten 27 aufweist. Beim Befüllen des Balgbeutels 29 entfalten sich die Falten 27, so dass sich deren Öffnungswinkel α vergrößert, im Extremfall bis auf 180°, wobei sich die Länge des Balgbeutels vergrößert. Um aus dem Balgbeutel 29 Flüssigkeit zu entnehmen, wird dieser mit der Pumpenstange 31 zusammen geschoben. Dabei verkürzt sich der Balgbeutel und der Öffnungswinkel α der Falten 27 reduziert sich, bis im Extremfall die Falten 27 vollständig geschlossen ist, also der Öffnungswinkel α auf null reduziert ist. Figur 10 ist bezüglich der Darstellung der Falten 27 idealisiert, um das Prinzip zu veranschaulichen. Bei realen Balgbeuteln sind die Falten 27 weniger scharf ausgeprägt und können im Extremfall die Form von Wellen annehmen, so dass das Erscheinungsbild eines Balgbeutels auch einem Wellrohr ähneln kann.

Balgbeutel sind zur Lagerung von flüssigen Medikamenten, beispielsweise Augentropfen, an sich bekannt und können mit so genannten Blow-Fill-Seal Verfahren kostengünstig hergestellt werden.

Der in Figur 10 dargestellte Balgbeutel 29 ist aus Polyethylen gefertigt. Zur Ankopplung an die Pumpenstange 31 weist er ein Kopplungselement in Form eines hinterschnittenen Kopfs 41 auf, der in eine passende Aufnahme der Pumpenstange 31 eingelegt wird.

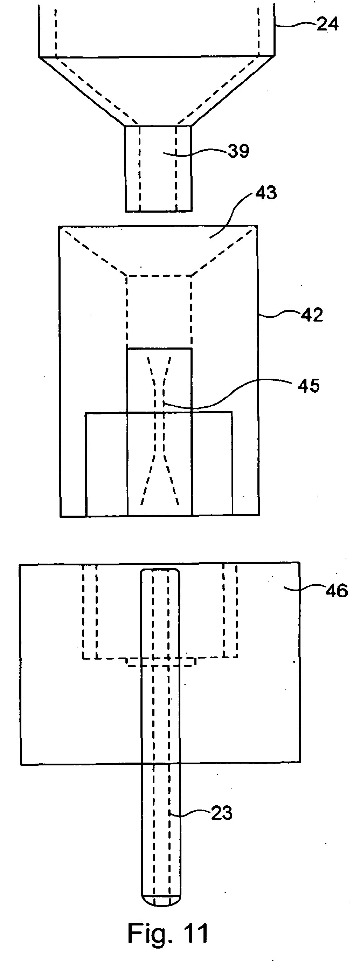

Bei dem in den Figuren 4 und 6 dargestellten Ausführungsbeispiel wird das Speichervolumen 7 der Pumpeneinheit 4, das beispielsweise ein eingesetzter Medikamentenbehälter oder ein fest eingebauter Tank sein kann, mittels einer Transferkanüle 26 bzw. 33, die ein Septum 25 durchsticht, an die Flüssigkeitsleitung 23 bzw. 34 der Kanülenträgereinheit 1 bzw. der Ladestation 9 angeschlossen. In Figur 11 ist eine alternative Anschlussmöglichkeit dargestellt. Die Öffnung 39 des Medikamentenbehälters 24 bzw. eines eingebauten Tanks ist als ein Rohrabschnitt ausgebildet, auf den ein Kupplungsstück 42 mit einem Ende aufgesteckt wird. Das Kupplungsstück 42 ist maßgenau an den Außendurchmesser dieses Rohrabschnitts angepasst, so dass sich eine dichte Verbindung ergibt. Zusätzlich hat das Kupplungsstück 42 eine trichterförmige Ausnehmung 43, die zu einem zylinderförmigen Ansatz 44 des Medikamentenbehälters 24 bzw. Tanks passt, so dass das Kupplungsstück mit der Innenfläche der Ausnehmung 43 flächig an dem Ansatz 44, dessen Mitte in den Rohrabschnitt übergeht, anliegt und auf diese Weise die mechanische Belastbarkeit der Verbindung mit dem Kupplungsstück erhöht.

Das Kupplungsstück 42 enthält ein Verbindungsstück 45 aus einem elastomeren Werkstoff, wie er im Anwendungsbereich steriler Flüssigkeiten typischerweise verwendet wird. Das Verbindungsstück 45 hat zwei trichterförmige Öffnungen. Innen befindet sich ein Längskanal, der erst durch Hineinschieben der kapillaren Flüssigkeitsleitung 23 geöffnet wird. Das Verbindungsstück 45 liegt dann an der Öffnung 39 des Medikamentenbehälters 24 an. Auf diese Weise wird die Hygiene beim Lösen und Wieder-Verbinden verbessert. Als zusätzliche Maßnahme zur Verbesserung der Zuverlässigkeit der Verbindung kann die Flüssigkeitsleitung 23 mit einem Endstück 46 versehen werden, das mit dem Kupplungsstück 42 eine Steckverbindung bildet.

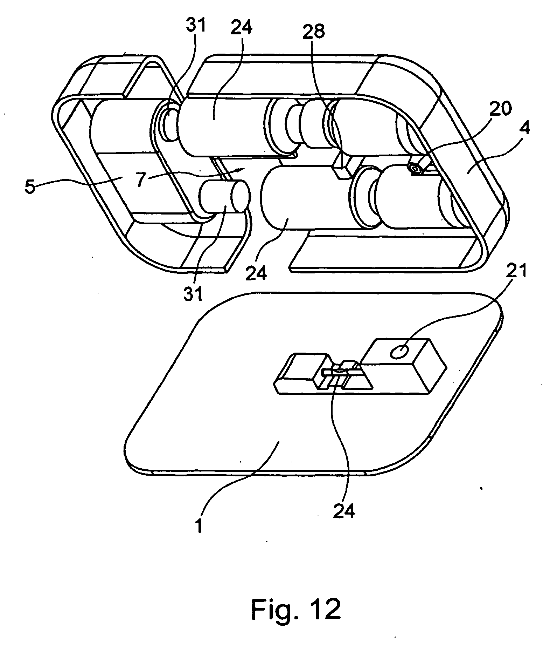

Figur 12 zeigt ein weiteres Ausführungsbeispiel einer Pumpeneinheit 4 mit einem dazu passenden Ausführungsbeispiel einer Kanülenträgereinheit 1. Die Pumpeneinheit 4 kann an der Kanülenträgereinheit 1 mittels Verbindungselementen 20, 21 lösbar befestigt werden. Die Verbindungselemente 20, 21 bewirken bei dem dargestellten Ausführungsbeispiel eine kraftschlüssige Verbindung. Ein Verbindungselement 20 wird hierfür von einem konischer Zapfen gebildet, der in eine Buchse, die das korrespondierende Verbindungselement 21 bildet eingreift. Der wesentliche Unterschied der in Figur 9 dargestellten Pumpeneinheit 4 zu den im vorhergehenden beschriebenen Ausführungsbeispielen besteht darin, dass das Speichervolumen 7 der Pumpeneinheit 4 zur Aufnahme von zwei Medikamentenampullen 24 ausgebildet ist. Die Pumpeneinheit 4 hat für jede der beiden Medikamentenampullen 24 einen Pumpenkolben 31, der zum Auspressen von in den Medikamentenampullen 24 enthaltener Flüssigkeit in diese eingeführt wird.

Die Pumpeneinheit 4 enthält eine Flüssigkeitsleitung, die über zwei Zweige einen Anschluss der Ampullen 24 an die Infusionskanüle der Kanülenträgereinheit 1 ermöglicht (nicht dargestellt). Im Betrieb wird von der Pumpeneinheit 4 zunächst nur aus einer der beiden Medikamentenampullen 24 Flüssigkeit ausgepresst. Erst wenn diese leer ist, wird Flüssigkeit aus der zweiten Medikamentenampulle 24 entnommen. Wenn eine eingesetzte Medikamentenampulle 24 leer ist, erzeugt die Pumpeneinheit 4 ein Signal, um diesen Zustand einem Benutzer anzuzeigen, damit die leere Medikamentenampulle 24 austauschen kann, bevor auch die zweite Medikamentenampulle 24 leer ist.

Bei diesem Ausführungsbeispiel kann auf eine Ladestation 9 prinzipiell verzichtet werden, sofern in der Pumpeneinheit ein Energiespeicher mit einer ausreichenden Speicherkapazität vorhanden ist. Der Einsatz von Akkumulatoren und einer Ladestation 9 ist jedoch auch für dieses Ausführungsbeispiel vorteilhaft und deshalb bevorzugt. Insbesondere reicht eine einzige Pumpeneinheit 4 prinzipiell für einen Patienten aus, da dieser bei Bedarf jeweils eine der beiden Medikamentenampullen in der Pumpeneinheit ersetzt. Durch eine Pumpeneinheit 4 mit einer Aufnahme für zwei Medikamentenampullen 24 wird es einem Patienten ermöglicht, den Zeitpunkt eines Ampullenwechsels innerhalb einem komfortablen Zeitfenster von mehreren Stunden frei zu wählen, ohne dass wertvoller Inhalt der Medikamentenampullen 24 verschwendet wird.

Ein Aspekt der Erfindung, der auch selbstständige Bedeutung haben kann, betrifft deshalb Infusionssystem zum Verabreichen eines flüssigen Medikaments, insbesondere Insulin, mit einer an eine Infusionskanüle anschließbaren Pumpeneinheit 4, die eine Pumpe 5, einen elektrischen Energiespeicher zur Energieversorgung der Pumpe 5 und zwei Aufnahmen, in die jeweils eine Ampulle 24 mit dem zu verabreichende Medikament einsetzbar ist, umfasst.

### Bezugszahlenliste:

- 1.: Kanülenträgereinheit
- 2.: Infusionskanüle
- 3.: Auflagefläche der Kanülenträgereinheit
- 4.: Pumpeneinheit
- 5.: Pumpe
- 6.: Akkumulator
- 7.: Speichervolumen
- 8.: Gehäuse der Pumpeneinheit
- 9.: Ladestation
- 10.: Aufnahmefach
- 11.: Pumpe der Ladestation
- 12.: Energiespeicher
- 13.: Steckverbinder
- 14.: Messgerät
- 15.: Anzeigeeinrichtung
- 16.: Bedienungselemente
- 17.: Sender
- 18.: Empfänger
- 19.: -
- 20.: Verbindungselement
- 21.: Verbindungselement
- 22.: Dichtung
- 23.: Flüssigkeitsleitung
- 24.: Medikamentenampulle
- 25.: Septum
- 26.: Transferkanüle
- 27.: Falten
- 28.: Drucksensor
- 29.: Balgbeutel
- 30.: Steuerungselektronik
- 31.: Pumpenkolben
- 32.: Vorratsbehälter
- 33.: Transferkanüle
- 34.: Flüssigkeitsleitung
- 35.: Septum
- 36.: Transferkanüle
- 37.: Stopfen
- 38.: Balg
- 39.: Ampullenöffnung
- 40.: Transfermodul
- 41.: Kopf
- 42.: Kupplungsstück
- 43.: Ausnehmung
- 44.: Ansatz
- 45.: Kapillarrohr
- 46.: Endstück

## Patentansprüche

1. Infusionssystem zum Verabreichen eines flüssigen Medikaments, insbesondere Insulin, mit
einer Infusionskanüle (2) zum Einstich in den Körper des Patienten,
einer Pumpeneinheit (4), die eine Pumpe (5), einen Akkumulator (6) zur Energieversorgung der Pumpe (5) und ein Speichervolumen (7) für das zu verabreichende Medikament umfasst,
einer Ladestation (9) für die Pumpeneinheit (4) zum Aufladen des Akkumulators (6) der Pumpeneinheit (4), eine Kanülenträgereinheit (1), die bestimmungsgemäß am Körper eines Patienten befestigt wird, die Infusionskanüle (2) zum Einstich in den Körper des Patienten aufweist, und die Kanülenträgereinheit (1) und die Pumpeneinheit (4) derart aneinander angepasst sind, dass nach einem Anschließen der Pumpeneinheit (4) an die Kanülenträgereinheit (1) die Pumpeneinheit (4) an der Kanülenträgereinheit (1) anliegt,
**dadurch gekennzeichnet, dass**
die Ladestation (9) zum Auffüllen des Speichervolumens (7) mit dem zu verabreichenden Medikament eingerichtet ist.

2. Infusionssystem nach Anspruch 1, **dadurch gekennzeichnet, dass** das Speichervolumen (7) zur Aufnahme von mindestens einem Medikamentenbehälter (24, 29), insbesondere zur Aufnahme von zwei Medikamentenbehältern (24, 29), ausgebildet ist.

3. Infusionssystem nach einem der vorhergehenden Ansprüche, **gekennzeichnet durch** zwei Pumpeneinheiten, die bestimmungsgemäß abwechselnd in der Ladestation (9) aufgeladen werden.

4. Infusionssystem nach einem der vorhergehenden Ansprüche, **dadurch gekennzeichnet, dass** die Ladestation (9) ein Messgerät (14) zur Bestimmung einer Analytkonzentration, insbesondere des Glukosegehalts, einer Körperflüssigkeitsprobe aufweist.

5. Infusionssystem nach einem der vorhergehenden Ansprüche, **dadurch gekennzeichnet, dass** die Ladestation (9) und die Pumpeneinheit (4) Mittel (17, 18) zur Übertragung von Daten aufweisen.

6. Infusionssystem nach einem der vorhergehenden Ansprüche, **dadurch gekennzeichnet, dass** die Pumpeneinheit (4) einen Drucksensor (28) zum Überwachen eines Infusionsdrucks des flüssigen Medikamentes aufweist.

7. Infusionssystem nach einem der vorhergehenden Ansprüche, **dadurch gekennzeichnet, dass** der Akkumulator (6) für einen Benutzer unzugänglich in der Pumpeneinheit (4) eingebaut ist.

8. Infusionssystem nach einem der vorhergehenden Ansprüche, **dadurch gekennzeichnet, dass** die Ladestation (9) dafür eingerichtet ist, die Energie zum Aufladen des Akkumulators (6) einer angekoppelten Pumpeneinheit (4) einem internen Energiespeicher (12) zu entnehmen.

9. Infusionssystem nach einem der vorhergehenden Ansprüche, **dadurch gekennzeichnet, dass** die Pumpeneinheit (4) einen Sensor zum Überwachen des Füllstands des Speichervolumens (7) aufweist.

10. Infusionssystem nach einem der vorhergehenden Ansprüche, **gekennzeichnet durch** zwei Pumpeneinheiten (4), die jeweils eine Pumpe (5) und ein Speichervolumen (7) für das zu verabreichende Medikament umfassen und bestimmungsgemäß abwechselnd in der Ladestation (9) aufgeladen werden.

11. Infusionssystem nach einem der vorhergehenden Ansprüche, **gekennzeichnet durch** einen auffüllbarer Balgbeutel (29), der ein vorderes Ende mit einer Öffnung (39) zum Durchtritt des Medikaments und ein hinteres Ende mit einem Kopplungselement (41) zum formschlüssigen Ankoppeln an eine Pumpenstange (31) aufweist.

12. Infusionssystem nach einem der vorhergehenden Ansprüche, **gekennzeichnet durch** eine auffüllbare Ampulle zur Speicherung eines flüssigen Medikaments, wobei die Ampulle (24) ein vorderes Ende mit einer Öffnung (39) zum Durchtritt des Medikaments und ein hinteres Ende, das mit einem verschiebbaren Stopfen (37) verschlossen ist, aufweist, **dadurch** gekennzeichnet, dass an dem Stopfen (37) ein Balg (38) befestigt ist, der die Innenwände der Ampulle (24) vor Kontamination schützt, wenn der Stopfen (37) in die Ampulle (24) hinein geschoben ist

13. Infusionssystem nach einem der vorhergehenden Ansprüche, **dadurch gekennzeichnet, dass** das Speichervolumen (7) der Pumpeneinheit (4) zur Aufnahme von mindestens zwei Medikamentenbehältern (24) ausgebildet ist.

14. Pumpeneinheit für ein Infusionssystem nach einem der vorstehenden Ansprüche, **dadurch gekennzeichnet, dass** die Pumpeneinheit (4) eine Pumpe (5), einen Akkumulator (6) zur Energieversorgung der Pumpe (5) und ein Speichervolumen (7) für das zu verabreichende Medikament umfasst und die Ladestation (9) des Infusionssystems derart angepasst ist, dass mittels der Ladestation (9) sowohl der Akkumulator (6) als auch das Speichervolumen (7) aufladbar ist, wobei die Pumpeneinheit (4) derart an eine Kanülenträgereinheit (1) angepasst ist, dass nach einem Anschließen der Pumpeneinheit (4) an die Kanülenträgereinheit (1) die Pumpeneinheit (4) an der Kanülenträgereinheit (1) anliegt.

## Claims

1. Infusion system for administering a liquid medicinal agent, in particular insulin, comprising
an infusion cannula (2) for puncturing into the body of the patient;
a pump unit (4) comprising a pump (5), a rechargeable battery (6) for supplying energy to the pump (5), and a storage volume (7) for the medicinal agent to be administered;
a charging station (9) for the pump unit (4) for charging the rechargeable battery (6) of the pump unit (4),
a cannula carrier unit (1), which, according to its purpose, is to be secured to the body of a patient, comprises the infusion cannula (2) for puncturing into the body of the patient, and
the cannula carrier unit (1) and the pump unit (4) are adapted to each other so that the pump unit (4) rests against the cannula carrier unit (1) after the pump unit (4) has been connected to the cannula carrier unit (1),
**characterized in that**
the charging station (9) is set up for filling the storage volume (7) with the medicinal agent to be administered.

2. Infusion system according to claim 1, **characterized in that** the storage volume (7) is provided for receiving at least one medicinal agent container (24, 29), in particular for receiving two medicinal agent containers (24, 29).

3. Infusion system according to any one of the preceding claims, **characterized by** two pump units, which, according to purpose, are charged in the charging station (9) in an alternating manner.

4. Infusion system according to any one of the preceding claims, **characterized in that** the charging station (9) comprises a measuring device (14) for determining an analyte concentration, in particular the glucose content, of a sample of body fluid.

5. Infusion system according to any one of the preceding claims, **characterized in that** the charging station (9) and the pump unit (4) comprise means (17, 18) for transmitting data.

6. Infusion system according to any one of the preceding claims, **characterized in that** the pump unit (4) has a pressure sensor (28) for monitoring an infusion pressure of the liquid medicinal agent.

7. Infusion system according to any one of the preceding claims, **characterized in that** the rechargeable battery (6) is incorporated into the pump unit (4) such as to be inaccessible for a user.

8. Infusion system according to any one of the preceding claims, **characterized in that** the charging station (9) is set up to take the energy for charging the rechargeable battery (6) of a pump unit (4) coupled to it from an internal energy storing facility (12).

9. Infusion system according to any one of the preceding claims, **characterized in that** the pump unit (4) has a sensor for monitoring the filling level of the storage volume (7).

10. Infusion system according to any one of the preceding claims, **characterized by** two pump units (4), which each comprise a pump (5) and a storage volume (7) for the medicinal agent to be administered and, according to their purpose, are charged in the charging station (9) in an alternating manner.

11. Infusion system according to any one of the preceding claims, **characterized by** a refillable bellows bag comprising a front end with an opening (39) for passage of the medicinal agent and a back end with a coupling element (41) for positive-fit coupling to a pump rod (31).

12. Infusion system according to any one of the preceding claims, **characterized by** a refillable vial for storing a liquid medicinal agent, whereby the vial (24) has a front end with an opening (39) for passage of the medicinal agent and a back end that is closed off by a shiftable plug (37), **characterized in that** the a bellows (38) is secured to the plug (37) and protects the internal walls of the vial (24) from contamination when the plug (37) is being pushed into the vial (24).

13. Infusion system according to any one of the preceding claims, **characterized in that** the storage volume (7) of the pump unit (4) is configured for receiving at least two medicinal agent containers (24)

14. Pump unit for an infusion system according to any one of the preceding claims, whereby the pump unit (4) comprises a pump (5), a rechargeable battery (6) for supplying energy to the pump (5), and a storage volume (7) for the medicinal agent to be administered and is adapted to a cannula carrier unit (1) of the infusion system such that the pump unit (4) rests against the cannula carrier unit (1) after attachment of the pump unit (4) to the cannula carrier unit (1).

## Revendications

1. Système de perfusion servant à administrer un médicament liquide, notamment de l'insuline, comprenant
une canule de perfusion (2) destinée à être introduite dans le corps du patient,
une unité de pompe (4) qui comprend une pompe (5), un accumulateur (6) pour l'alimentation en énergie de la pompe (5) et un volume de stockage (7) pour le médicament à administrer,
une station de charge (9) pour l'unité de pompe (4), permettant de recharger l'accumulateur (6) de l'unité de pompe (4),
une unité de support de canule (1), prévue pour être fixée sur le corps d'un patient et qui comporte la canule de perfusion (2), destinée à être introduite dans le corps du patient,
l'unité de support de canule (1) et l'unité de pompe (4) étant adaptées l'une à l'autre de sorte que l'unité de pompe (4) est appliquée contre l'unité de support de canule (1) après avoir raccordé l'unité de pompe (4) à l'unité de support de canule (1),
**caractérisé en ce que**
la station de charge (9) est conçue pour remplir le volume de stockage (7) avec le médicament à administrer.

2. Système de perfusion selon la revendication 1, **caractérisé en ce que** le volume de stockage (7) est conçu pour recevoir au moins un récipient pour médicament (24, 29), en particulier pour recevoir deux récipients pour médicament (24, 29).

3. Système de perfusion selon l'une des revendications précédentes, **caractérisé par** deux unités de pompe, prévues pour être rechargées de façon alternée dans la station de charge (9).

4. Système de perfusion selon l'une des revendications précédentes, **caractérisé en ce que** la station de charge (9) comporte un appareil de mesure (14) pour déterminer une concentration d'analyte, en particulier le taux de glucose, d'un échantillon de liquide organique.

5. Système de perfusion selon l'une des revendications précédentes, **caractérisé en ce que** la station de charge (9) et l'unité de pompe (4) comportent des moyens (17, 18) de transmission de données.

6. Système de perfusion selon l'une des revendications précédentes, **caractérisé en ce que** l'unité de pompe (4) comporte un capteur de pression (28) pour surveiller une pression de perfusion du médicament liquide.

7. Système de perfusion selon l'une des revendications précédentes, **caractérisé en ce que** l'accumulateur (6) est monté dans l'unité de pompe (4) de manière à être inaccessible pour un utilisateur.

8. Système de perfusion selon l'une des revendications précédentes, **caractérisé en ce que** la station de charge (9) est conçue pour prélever l'énergie permettant de recharger l'accumulateur (6) d'une unité de pompe (4) accouplée dans un réservoir d'énergie (12) interne.

9. Système de perfusion selon l'une des revendications précédentes, **caractérisé en ce que** l'unité de pompe (4) comporte un capteur pour surveiller le niveau de remplissage du volume de stockage (7).

10. Système de perfusion selon l'une des revendications précédentes, **caractérisé par** deux unités de pompe (4), qui comprennent chacune une pompe (5) et un volume de stockage (7) pour le médicament à administrer et qui sont prévues pour être rechargées de manière alternée dans la station de charge (9).

11. Système de perfusion selon l'une des revendications précédentes, **caractérisé par** une poche à soufflet (29) gonflable, qui présente une extrémité avant pourvue d'une ouverture (39) pour le passage du médicament et une extrémité arrière pourvue d'un élément de couplage (41) pour le couplage par complémentarité de forme à une tige de pompe (31).

12. Système de perfusion selon l'une des revendications précédentes, **caractérisé par** une ampoule rechargeable pour le stockage d'un médicament liquide, ladite ampoule (24) présentant une extrémité avant pourvue d'une ouverture (39) pour le passage du médicament et une extrémité arrière fermée par un bouchon (37) déplaçable, **caractérisé en ce qu'**un soufflet (38) est fixé au bouchon (37), soufflet qui protège les parois internes de l'ampoule (24) de toute contamination lorsque le bouchon (37) est introduit dans l'ampoule (24).

13. Système de perfusion selon l'une des revendications précédentes, **caractérisé en ce que** le volume de stockage (7) de l'unité de pompe (4) est conçu pour recevoir au moins deux récipients pour médicament (24).

14. Unité de pompe pour un système de perfusion selon l'une des revendications précédentes, **caractérisée en ce que** l'unité de pompe (4) comprend une pompe (5), un accumulateur (6) pour l'alimentation en énergie de la pompe (5) et un volume de stockage (7) pour le médicament à administrer et la station de charge (9) du système de perfusion est adaptée de sorte à pouvoir recharger au moyen de ladite station de charge (9) l'accumulateur (6) ainsi que le volume de stockage (7), l'unité de pompe (4) étant adaptée à une unité de support de canule (1) de sorte que l'unité de pompe (4) est appliquée contre l'unité de support de canule (1) après avoir raccordé l'unité de pompe (4) à l'unité de support de canule (1).
